# EUROPÄISCHE PATENTSCHRIFT

(11) **EP 0 867 426 B1**
(45) Veröffentlichungstag und Bekanntmachung des Hinweises auf die Patenterteilung: **26.06.2002**
(21) Anmeldenummer: 98110087.8
(22) Anmeldetag: 05.09.1994
(51) Int. Cl.: C07C 59/265, C07C 51/42

(54) **Modifizierte Kristalle einer festen, essbaren, organischen Säure**
Modified crystals of a solid edible organic acid
Cristaux modifiés d'un acide organique comestible solide

(30) Priorität: 07.09.1993 CH 265293
(43) Veröffentlichungstag der Anmeldung: 30.09.1998
(62) Teilanmeldung aus: 94113877.8
(73) Patentinhaber: Gergely, Gerhard, Dr., A-1053 Wien (AT)
(72) Erfinder: Gergely, Gerhard, Dr., 1053 Wien (AT); Gergely, Irmgard, 1053 Wien (AT); Gergely, Thomas, Dr., 1053 Wien (AT); Gergely, Stefan, Dr., 1050 Wien (AT)
(74) Vertreter: Büchel, Kurt F., Dr.

(56) Entgegenhaltungen:
- EP-A- 0 076 340
- WO-A-88/00009
- WO-A-93/00886
- CH-A- 662 926

## Beschreibung

Die Oberflächenpassivierung von Kristallen einer festen, essbaren, organischen Säure, insbesondere Zitronensäure, kann in an sich bekannter Weise, z.B. wie in der EP-B1-272 312 oder WO93/00886 beschrieben, durchgeführt werden. Erfindungsgemäß wird die Umsetzung zwischen Säure und Carbonat bei der Passivierung umgangen, indem man die Oberfläche der Säurekristalle, z.B. der Zitronensäure, mit einer Lösung von Natriumcitrat überzieht bzw. abdeckt. Auf diese Schicht kann anschliessend wiederum Natriumbicarbonat und/oder Natriumcarbonat - gegebenenfalls aus einer Lösung - in Schichtform aufgebracht werden. Dieses Aufbringen erfolgt besonders zweckmässig in einem Vakuummischkessel und gibt den bestmöglichen Schutz.

## Patentansprüche

1. Modifizierte Kristalle einer festen, essbaren, organischen Säure, insbesondere Zitronensäure, **dadurch gekennzeichnet, dass** sie von einer Carbonatfreien Schicht von Natriumcitrat überzogen und getrocknet sind.

## Claims

1. Modified crystals of a solid, edible, organic acid, particularly citric acid, **characterised in that** they are covered with a layer of sodium citrate which is free of carbonate and are dried.

## Revendications

1. Cristaux modifiés d'un acide organique alimentaire solide, en particulier de l'acide citrique, **caractérisés en ce qu'**ils sont couverts d'une couche de citrate de sodium exempt de carbonate et qu'ils sont séchés
